# FASCICULE DE BREVET EUROPEEN

(11) **EP 0 609 123 B1**
(45) Date de publication et mention de la délivrance du brevet: **06.12.1995**
(21) Numéro de dépôt: 94400139.5
(22) Date de dépôt: 24.01.1994
(51) Int. Cl.: C07C 17/20, C07C 17/00, C07C 19/12

(54) **Procédé de fluoration du perchloréthylène ou du pentachloréthane**
Verfahren zur Fluorierung von Perchlorethylen oder Pentachlorethan
Process for the fluorination of perchloroethylene or pentachloroethane

(30) Priorité: 27.01.1993 FR 9300779
(43) Date de publication de la demande: 03.08.1994
(73) Titulaire: ELF ATOCHEM S.A., 92800 Puteaux (FR)
(72) Inventeur: Cheminal, Bernard, F-69510 Soucieu-en-Jarrest (FR); Lacroix, Eric, F-69480 AMBERIEUX D AZERGUES (FR); Lantz, André, F-69390 Vernaison (FR)
(74) Mandataire: Leboulenger, Jean

(56) Documents cités:
- EP-A- 0 298 662
- EP-A- 0 328 127
- EP-A- 0 486 333

## Description

La présente invention concerne un procédé continu de fluoration du perchloréthylène ou du pentachloréthane et a plus particulièrement pour objet la fluoration de ces composés en phase gazeuse au moyen d'acide fluorhydrique en présence d'un catalyseur.

Les composés désirés (globalement désignés ci-après par l'expression "série F120") sont le F121 (CHCl₂-CCl₂F), le F122 (CHCl₂-CClF₂), le F123 (CHCl₂-CF₃), le F124 (CHClF-CF₃), le F125 (CHF₂-CF₃) ou leurs isomères qui pouvent être utilisés soit comme substituts des perfluorocarbures (C.F.C.) dans les domaines des mousses (agents gonflants et isolants), des aérosols (agents propulseurs) ou dans la réfrigération, soit comme intermédiaires de synthèse de ces substituts. On recherche actuellement des procédés performants pour leur production industrielle, et plus particulièrement pour celle du F124 et du F125.

Le brevet FR 1315351 décrit un procédé de fluoration en phase gazeuse d'halo-oléfines avec un catalyseur préparé par fluoration partielle d'une alumine imprégnée d'un ou plusieurs halogénures de métaux polyvalents comme le chrome, nickel, cobalt, manganèse, ... Les auteurs insistent sur la fluoration partielle du catalyseur car il s'avère, dans leur cas, qu'une fluoration poussée (>80 %) dudit catalyseur se traduit par une perte rapide de l'activité. Les essais de l'exemple 4 décrivant la fluoration du perchloréthylène sont réalisés avec un catalyseur à base de dérivés du chrome et du cobalt déposés sur Al₂O₃. A basse température (T<290°C), les inventeurs obtiennent malgré un rapport molaire HF/C₂Cl₄ de 5, un mélange de F121, F122, F123 et des oléfines F1111 (CFCl=CCl₂) et F1112a (CF₂=CCl₂). Pour ce mélange, il est noté que le F123 est nettement majoritaire mais apparemment les auteurs n'observent pas la formation de F124, F124a (CF₂Cl-CHF₂) et F125. Par ailleurs, on constate que les sélectivités en oléfines sont déjà importantes à basse température et croîssent avec celle-ci.

La demande de brevet WO 8911467 revendique la préparation du F123 et/ou du F124 par fluoration en phase gazeuse du perchloréthylène par HF en présence d'un catalyseur comprenant un métal -à un degré d'oxydation supérieur à zéro- choisi dans le groupe constitué par Cr, Co, Mn, Rh, Ni sur un support d'alumine, hautement fluoré. Contrairement au brevet précédent, il apparaît nécessaire de fluorer fortement le catalyseur (AlF₃ > 90 %) avant de procéder à la fluoration des produits organiques. L'objectif du procédé revendiqué est de produire du F123 et du F124 en minimisant la formation de F125. Le métal préféré des auteurs est le cobalt; cependant, d'après les exemples, on constate que, quelque soit le métal testé: Cr, Co, Mn ou Ni et malgré des températures et des rapports molaires HF/C₂Cl₄ élevés, les sélectivités en F125 restent très faibles (<10 %). Par ailieurs, il se forme de nombreux sous-produits (hors série F120); en particulier avec le chrome ou le nickel, les sélectivités en F123 + F124 + F125 n'excèdent pas 85-87 % lorsque l'on travaille à 350°C.

La demande de brevet JP 2-178237 décrit la fluoration du perchloréthylène en phase gazeuse sur des catalyseurs massiques constitués d'oxydes de chrome et d'au moins un des métaux suivants : Al, Mg, Ca, Ba, Sr, Fe, Ni, Co et Mn. Malgré des températures assez élevées (350-380°C), les catalyseurs massiques exemplifiés (co-métal Al, Mg, Ca, Ba, Sr ou Fe) conduisent à des sélectivités en F122 importantes, alors que celles en F125 restent inférieures à 15 %. Or, comme indiqué dans la demande de brevet FR 2661906, le F122 n'est pas un intermédiaire très intéressant pour la synthèse de F123-F124-F125 car il génère de nombreux sous-produits, notamment des oléfines.

La demande de brevet EP 366797 revendique un procédé de fluoration de composés saturés ou insaturés en présence d'un catalyseur à base d'alumine très pure (contenant moins de 100 ppm de sodium) et poreuse, servant de support à des fluorures métalliques (nickel, cobalt, fer, chrome, manganèse, cuivre ou argent). Cette technique qui exige un catalyseur de haute pureté est illustrée pour la série F120 (pentahalogénoéthanes) par deux exemples (27 et 30) avec un catalyseur à base de chrome supporté sur alumine. Dans un cas le F123 est obtenu majoritairement, mais avec de faibles rendements (20 %), par fluoration de C₂Cl₄ en phase gazeuse; dans l'autre cas, suivant la température de réaction, c'est le F124 ou le F125 qui est majoritaire, mais cette fois la réaction de base est la fluoration (addition d'HF, puis échange Cl/F) du F1113 (CClF=CF₂).

La demande de brevet FR 2661906 concerne la synthèse du F124 et du F125 par fluoration en phase gazeuse du F123 avec un catalyseur à base de chrome déposé sur charbon actif. Sur ce type de catalyseur, le F123 génère peu de sous-produits non valorisables tels que les composés F115 (CF₃-CF₂Cl), F114a (CF₃-CFCl₂), F114 (CF₂Cl-CF₂Cl) et F133a (CF₃-CH₂Cl) ou pouvant nuire à la durée de vie du catalyseur telle que l'oléfine F1111. Par contre, dans ce même brevet, il est indiqué que les composés sous-fluorés du F123 (F122, F121, C₂Cl₄... ) ne sont pas de bons précurseurs pour le F124 et le F125.

Cette observation est confirmée par le brevet US 3258500 qui revendique l'utilisation de chrome, massique ou supporté sur alumine, pour des réactions de fluoration en phase gazeuse. Ainsi, l'exemple 17 (colonne 14) décrit la fluoration du perchloréthylène à 400°C avec un rapport molaire HF/C₂Cl₄ de 6,2/1 ; dans ce cas, la sélectivité en F123+F124+F125 est faible (47,7 %). Une diminution de la température de réaction (300°C) porte seulement cette sélectivité F123+F124+F125 à 79,7 % et dans ce cas le F125 n' est plus le composé majoritaire.

La demande de brevet FR 2669022 décrit l'utilisation d'un catalyseur à base de nickel et de chrome supportés sur AlF₃ ou alumine fluorée pour la fluoration spécifique du F133a (CF₃-CH₂Cl) en F134a (CF₃-CH₂F), ce catalyseur permettant d'obtenir de très bonnes sélectivités en F134a.

Il a maintenant été trouvé que ce type de catalyseur convient très bien pour la préparation des composés de la série F120, et plus particulièrement celle des composés F123, F124 et F125, à partir du perchloréthylène ou du pentachloréthane. Avec ce catalyseur, on peut en effet obtenir à la fois un taux de conversion très élevé (95 % ou plus) du composé de départ et une excellente sélectivité totale en F123 + F124 + F125 (de l'ordre de 90 % ou plus).

L'invention a donc pour objet un procédé continu de fluoration catalytique du perchloréthylène ou du pentachloréthane en phase gazeuse, au moyen d'acide fluorhydrique, caractérisé en ce que l'on utilise un catalyseur mixte composé d'oxydes, halogénures et/ou oxyhalogénures de nickel et de chrome déposés sur un support constitué de fluorure d'aluminium ou d'un mélange de fluorure d'aluminium et d'alumine.

Ce catalyseur peut être préparé de façon connue en soi à partir d'une alumine activée. Celle-ci peut dans une première étape être transformée en fluorure d'aluminium ou en mélange de fluorure d'aluminium et d'alumine, par fluoration à l'aide d'air (ou d'un inerte tel l'azote) et d'acide fluorhydrique, le taux de transformation de l'alumine en fluorure d'aluminium dépendant essentiellement de la température à laquelle est effectuée la fluoration de l'alumine (en général entre 200 et 450°C, de préférence entre 250 et 400°C). Le support est ensuite imprégné à l'aide de solutions aqueuses de sels de chrome et de nickel ou à l'aide de solutions aqueuses d'acide chromique, de sel de nickel et d'un réducteur du chrome comme le méthanol.

Lorsque l'on utilise l'acide chromique (CrO₃) comme précurseur du chrome, on peut réduire ce chrome par tout moyen connu de l'homme de l'art (réducteur chimique, réduction thermique ...), sous réserve que la technique utilisée ne nuise pas aux propriétés du catalyseur et donc à son activité. Le réducteur chimique préféré est le méthanol.

Comme sels de chrome et de nickel, on préfère utiliser les chlorures, mais on peut aussi employer d'autres sels tels que, par exemple, les oxalates, formiates, acétates, nitrates et sulfates ou le bichromate de nickel pourvu que ces sels soient solubles dans la quantité d'eau susceptible d'être absorbée par le support.

Le catalyseur utilisé dans le procédé selon l'invention peut aussi être préparé par imprégnation directe de l'alumine par des solutions des composés de chrome et de nickel ci-dessus mentionnées. Dans ce cas, la transformation d'au moins une partie (70 % ou plus) de l'alumine en fluorure d'aluminium s'effectue lors de l'étape d'activation du catalyseur.

Les alumines activées à utiliser pour la préparation du catalyseur selon la présente invention sont des produits bien connus, disponibles dans le commerce. Elles sont généralement préparées par calcination d'hydrates d'alumine à une température comprise entre 300 et 800°C. Les alumines activées, utilisables dans le cadre de la présente invention, peuvent contenir des teneurs importantes (jusqu'à 1000 ppm) de sodium sans que cela nuise à l'activité catalytique.

Le catalyseur selon l'invention peut contenir en masse de 0,5 à 20 % de chrome et de 0,5 à 20 % de nickel et, de préférence, entre 2 et 10 % de chacun des métaux dans un rapport atomique nickel/chrome compris entre 0,5 et 5, de préférence voisin de 1.

Avant de pouvoir catalyser la réaction de fluoration du perchloréthylène ou du pentachloréthane, le catalyseur selon l'invention doit être conditionné, c'est-à-dire transformé en constituants actifs et stables (aux conditions réactionnelles) par une opération préalable dite d'activation.

Ce traitement peut être réalisé soit "in situ" (dans le réacteur de fluoration) ou bien dans un appareillage adéquat conçu pour résister aux conditions d'activation. Celle-ci comprend généralement les étapes suivantes :
- séchage à basse température (100 à 150°C, de préférence 110 à 130°C) en présence d'air ou d'azote,
- séchage à haute température (250 à 450°C, de préférence 300 à 350°C) sous azote ou sous air,
- fluoration à basse température (180 à 300°C, de préférence à 200°C environ) au moyen d'un mélange d'acide fluorhydrique et d'azote, en contrôlant la teneur en HF de façon que la température ne dépasse pas 300°C, et
- finition sous courant d'acide fluorhydrique pur ou dilué par de l'azote à une température pouvant aller jusqu'à 450°C.

Pendant cette opération, les précurseurs catalytiques (halogénures de nickel et de chrome, chromate, bichromate de nickel, oxyde de chrome) sont transformés en fluorures et/ou oxyfluorures correspondants, ce qui entraîne un dégagement d'eau et/ou d'acide chlorhydrique.

Cette activation contribue également à accroître la fluoration de l'alumine lorsque l'on a réalisé l'imprégnation sur un support déjà partiellement fluoré, ou lorsque l'on imprégne directement l'alumine, à la fluoration de cette dernière. Dans ce dernier cas, il est nécessaire de contrôler parfaitement la température (la fluoration de l'alumine est très exothermique) si l'on ne veut pas nuire aux caractéristiques physiques du catalyseur ; par ailleurs, les quantités d'eau générées sont nettement plus importantes.

L'analyse chimique des éléments (chrome, nickel, fluor, aluminium, oxygène), après activation, permet de vérifier la composition minérale du catalyseur selon l'invention.

Les conditions opératoires pour la synthèse des composés fluorés de la série F120 par fluoration du perchloréthylène ou du pentachloréthane en phase gazeuse à l'aide d'acide fluorhydrique, en présence du catalyseur selon l'invention, sont les suivantes :
**a)** La température de la réaction est généralement comprise entre 200 et 450°C et dépend du produit final recherché. Le F125 nécessite des températures plus élevées que le F124 et lui même que le F123. La température optimale est comprise entre 250 et 400°C.
**b)** Le temps de contact, calculé comme étant le temps de passage des gaz (dans les conditions réactionnelles) à travers le volume de catalyseur en vrac, est compris entre 3 et 100 secondes, et plus particulièrement, entre 5 et 30 secondes. Cependant, afin d'obtenir un bon compromis entre le taux de conversion du produit de départ et des productivités en produits finaux élevées, la meilleure plage est de 7 à 20 secondes.
**c)** Le rapport molaire HF/C₂Cl₄ (ou C₂HCl₅) peut aller de 1/1 à 20/1 et préférentiellement de 2/1 à 10/1. Là encore, le taux de conversion du composé organique de départ et la distribution des produits formés à l'intérieur de la série F120 dépendent du rapport molaire choisi, une augmentation de ce rapport molaire conduisant à une amélioration du taux de conversion global et à un déplacement des produits formés vers des composés plus fluorés (F124 et F125). Il faut cependant noter qu'un rapport molaire trop faible (inférieur à la stoechiométrie) augmente la formation de produits non recyclables (perhalogénoéthanes, tétrahalogénoéthanes et oléfines), ces dernières pouvant être néfastes à la stabilité de l'activité catalytique.
**d)** Les composés de la série F120 sous-fluorés (par rapport au produit désiré) peuvent être recyclés au réacteur. Cependant, des proportions trop élevées de certains d'entre eux (notamment F122 et F121) à l'alimentation du réacteur peuvent être à l'origine d'une désactivation du catalyseur par encrassement via la formation d'oléfines (F1111, F1112a ...).
**e)** Dans des conditions opératoires susceptibles d'encrasser le catalyseur, il peut être judicieux d'introduire avec les réactifs, de l'oxygène à faible teneur. Cette teneur peut varier selon les conditions opératoires entre 0,02 et 5 % par rapport aux réactifs organiques (pourcentage molaire). Cet oxydant a pour but de réagir avec les "lourds" qui sont à l'origine de l'encrassement du catalyseur.
**f)** La réaction selon l'invention peut être menée à la pression atmosphérique ou à une pression supérieure à cette dernière. Pour des raisons pratiques, on opérera généralement dans une zone allant de 0 à 25 bars relatifs.
**g)** Le catalyseur peut fonctionner soit en lit fluidisé, soit en lit fixe. Lorsque le composé organique de départ est le perchloréthylène, il est préférable -afin d'éliminer facilement les calories générées par l'addition d'HF sur la double liaison- de travailler en lit fluide, en réacteur tubulaire ou en lit fixe avec un taux important de recyclage de composés stables de la série F120 , dont la fluoration est peu exothermique (F123, F124 ..).
**h)** Lorsque les produits recherchés sont des composés hautement fluorés comme le F124 ou le F125, on peut envisager de scinder la réaction en deux étapes (deux réacteurs).
**i)** Les matériaux utilisés pour la construction de l'installation doivent être compatibles avec la présence d'hydracides tels que HCl et HF ; ils peuvent être choisis en "Hastelloy" ou en "Inconel" qui sont résistants aux milieux corrosifs contenant ces hydracides.

La mise en oeuvre du procédé selon l'invention permet d'obtenir des composés de la série F120 avec de très bonnes sélectivités en produits recherchés et/ou en produits recyclables. En effet, le type de catalyseur proposé permet d'accéder à ces composés de la série F120 en limitant la formation de produits non valorisables comme les perhalogénoéthanes et les tétrahalogénoéthanes ou la formation des oléfines qui résultent d'une déshydrohalogénation des composés de la série F120 et encrassent le catalyseur. La limitation des premiers permet d'améliorer la rentabilité de l'installation ; celle des seconds joue sur la durée de vie du catalyseur et sur l'ajout d'oxygène. Il faut noter que si cet oxydant a un rôle bénéfique sur l'encrassement du catalyseur, il peut avoir un rôle néfaste en augmentant la formation de composés perhalogènés non valorisables via la réaction de DEACON. Or, il a été constaté -malgré la présence de chrome dans le catalyseur (cf. Chemical week 1987, 24 Juin, page 18)- que l'association du chrome et du nickel oriente préférentiellement la consommation de cet oxydant -lorsqu'il est présent- vers l'oxydation des "lourds" et donc limite la réaction de DEACON et par là même la formation de perhalogénoéthanes non valorisables, qui ne peuvent que perturber la purification des bons produits (cas du F115 vis-à-vis du F125).

Finalement, le catalyseur selon l'invention permet d'obtenir directement à partir du perchloréthylène ou du pentachloréthane, en une seule passe et avec des productivités compatibles avec une production industrielle (rapport molaire HF/C₂Cl₄ ou C₂HCl₅ inférieure à 10 ; temps de contact inférieur à 20 s) des sélectivités en composés hautement fluorés de la série F120 (F124, F125) importantes (de l'ordre de 25 à 40 % pour chaque produit) et ceci avec peu de produits non valorisables (<10 %).

Les exemples suivants illustrent l'invention sans la limiter.

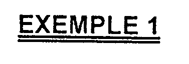

### 1A - PREPARATION ET ACTIVATION DU CATALYSEUR

Dans un évaporateur rotatif, on place 250 ml d'un support contenant en poids 73 % de fluorure d'aluminium et 27 % d'alumine, obtenu dans une étape précédente par fluoration d'alumine GRACE HSA en lit fluidisé vers 300°C à l'aide d'air et d'acide fluorhydrique (concentration volumique de 5 à 10 % de cet acide dans l'air). L'alumine GRACE HSA de départ présente les caractéristiques physicochimiques suivantes :
- forme : billes de 1-2 mm de diamètre
- surface BET : 220m²/g
- volume poreux : 1,2 cm³/g (pour les rayons de pores compris entre 4 nm et 63 »m)
- teneur en sodium : 600 ppm
On prépare par ailleurs deux solutions aqueuses séparées :
**(a)** solution chromique additionnée de chlorure de nickel contenant :

| | |
|---|---|
| - anhydride chromique | 12,5 g |
| - chlorure de nickel hexahydraté | 29 g |
| - eau | 42 g |

**(b)** Solution méthanolique contenant :

| | |
|---|---|
| - méthanol | 18 g |
| - eau | 50 g |

Le mélange de ces deux solutions est ensuite introduit, à température ambiante sous pression atmosphérique et en 45 minutes environ, sur le support en agitation. Le catalyseur est alors séché sous courant d'azote, en lit fluidisé, vers 110°C pendant 4 heures.

On charge 100 ml (77,5 g) de catalyseur sec dans un réacteur tubulaire en INCONEL de diamètre intérieur 27 mm et l'on monte la température à 120°C sous courant d'azote, à pression atmosphérique. On maintient ce traitement pendant une dizaine d'heures puis on remplace progressivement l'azote par de l'acide fluorhydrique en veillant à ce que l'augmentation de température n'excède pas 95°C et, lorsque l'on atteint un ratio molaire HF/N₂ de 50/50, on élève la température à 300°C.

Après disparition des pics d'exothermie, on monte la température à 350°C sous courant d'acide fluorhydrique pur (1 mole/heure) pendant 6 heures.

Le catalyseur est finalement balayé sous courant d'azote avant de démarrer le test catalytique. Les caractéristiques du catalyseur A ainsi séché et activé sont les suivantes :

| - composition chimique (pondérale) | |
|---|---|
| . fluor | 64,4 % |
| . aluminium | 27,2 % |
| . nickel | 3,75 % |
| . chrome | 3,3 % |
| . oxygène | 1,35 % |

| - propriétés physiques : | |
|---|---|
| . surface BET | 35,4 m²/g |
| . volume des pores d'un rayon compris entre 4 nm et 63 »m | 0,47 cm³/g |
| . surface des pores de rayon supérieur à 4 nm | 32,8 m²/g |

### 1B - FLUORATION DU PERCHLORETHYLENE

Les performances du catalyseur A dans la fluoration du perchloréthylène ont été testées à pression atmosphérique, sans addition d'oxygène, dans les conditions opératoires et avec les résultats rassemblés dans le tableau 1 suivant.

**TABLEAU 1**

| **TEST N°** | **11** | **12** |
|---|---|---|
| ***Conditions opératoires*** : | | |
| - Température (°C) | 350 | 300 |
| - Rapport molaire HF/C₂Cl₄ | 6,8 | 7,0 |
| -Temps de contact (s) | 15,9 | 16,7 |
| - Age du catalyseur (h) | 24 | 48 |

| ***Résultats*** : | | |
|---|---|---|
| - Taux de transformation global de C₂Cl₄ (%) | 96,0 | 98,3 |

| - Sélectivité (% molaire) en : | | |
|---|---|---|
| F125 | 23,4 | 1,9 |
| F124 | 32,9 | 27,1 |
| F124a | 0,4 | 0,6 |
| F123 | 38,9 | 68,7 |
| F123a | 0,2 | 0,2 |
| F122 | 0,2 | 0,4 |
| F121 | 0 | 0 |
| F133a | 1,3 | 0,3 |
| F115 | 1,0 | 0,1 |
| F114 + F 114a | 0,7 | 0,3 |
| F1111 | 0,7 | 0,3 |

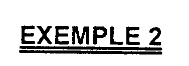

On prépare un catalyseur B selon l'invention dont les teneurs en chrome et en nickel sont sensiblement le double de celles du catalyseur A. On opère comme à l'exemple 1A, mais en utilisant les deux solutions aqueuses suivantes :
**(a)** solution chromique additionnée de chlorure de nickel contenant :

| | |
|---|---|
| - anhydride chromique | 25 g |
| - chlorure de nickel hexahydraté | 58 g |
| - eau | 40 g |

**(b)** Solution méthanolique contenant :

| | |
|---|---|
| - méthanol | 35 g |
| - eau | 30 g |

Après séchage et activation, les caractéristiques du catalyseur B sont les suivantes :

| - composition chimique (pondérale) | |
|---|---|
| . fluor | 58,5 % |
| . aluminium | 25,1 % |
| . nickel | 6,8 % |
| . chrome | 5,6 % |

| - propriétés physiques : | |
|---|---|
| . surface BET | 15,1 m²/g |
| . volume des pores d'un rayon compris entre 4 nm et 63 »m | 0,382 cm³/g |
| . surface des pores de rayon supérieur a 4 nm | 18 m²/g |

Le tableau 2 suivant rassemble les conditions opératoires et les résultats obtenus avec ce catalyseur B dans la fluoration du perchloréthylène à pression atmosphérique.

**TABLEAU 2**

| **TEST N°** | **21** | **22** | **23** | **24** |
|---|---|---|---|---|
| ***Conditions opératoires*** : | | | | |
| - Température (°C) | 350 | 350 | 330 | 300 |
| - Rapport molaire HF/C₂Cl₄ | 7,4 | 7,2 | 7,2 | 7,3 |
| -Temps de contact (s) | 15 | 15,5 | 16,5 | 16,8 |
| - Age du catalyseur (h) | 44 | 556 | 315 | 453 |

| ***Résultats*** : | | | | |
|---|---|---|---|---|
| - Taux de transformation global de C₂Cl₄ (%) | 95,6 | 94,6 | 95 | 94,1 |

| - Sélectivité (% molaire) en : | | | | |
|---|---|---|---|---|
| F125 | 32 | 34,1 | 25,5 | 8,3 |
| F124 | 28,4 | 24,2 | 31,3 | 33,5 |
| F124a | 0,4 | 0,3 | 0,3 | 0,6 |
| F123 | 33,3 | 33,1 | 39,3 | 54,6 |
| F123a | 0,2 | 0,3 | 0,2 | 0,4 |
| F122 | 0,2 | 0,3 | 0,3 | 0,6 |
| F121 | traces | 0 | 0 | 0 |
| F133a | 1,6 | 2,5 | 0,9 | 0,5 |
| F115 | 1,3 | 1,4 | 0,4 | 0,005 |
| F114 + F 114a | 1,2 | 2,2 | 0,8 | 0,5 |
| F1111 | 1,0 | 1,4 | 0,9 | 0,9 |

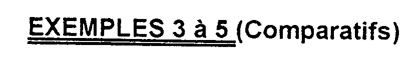

A titre comparatif, trois catalyseurs C, D et E non conformes à la présente invention ont été préparés de la façon suivante :

### Ex. 3 : Catalyseur C sans chrome

On opère comme à l'exemple 1A, mais en supprimant la solution méthanolique (b) et en remplaçant la solution chromique (a) par une solution de 59 g de chlorure de nickel hexahydraté dans 75 g d'eau.

### Ex. 4 : Catalyseur D sans nickel

On opère comme à l'exemple 1A, mais en remplaçant la solution (a) par une solution de 25 g d'anhydride chromique dans 54 g d'eau.

### Ex. 5 : Catalyseur E (Ni + Cr sur charbon)

Dans un évaporateur rotatif, on place 250 ml d'un support charbon actif végétal, préalablement séché à 150°C, présentant les caractéristiques physicochimiques suivantes :

| | |
|---|---|
| - densité apparente | 0,41 |
| - granulométrie | extrudés de 0,8 mm |
| - surface BET | 922 m²/g |
| - surface des pores de 5 à 25 nm | 20,4 m²/g |
| - surface des pores de 25 à 32 nm | 3,2 m²/g |

et l'imprègne avec une solution aqueuse contenant 13 g d'anhydride chromique, 29 g de chlorure de nickel hexahydraté et 62 g d'eau.

Le catalyseur est ensuite séché sous courant d'azote, en lit fluidisé, vers 110°C pendant 4 heures.

On charge 100 ml (50,4 g) de catalyseur sec dans un réacteur tubulaire et on procède à une activation identique à celle de l'exemple 1A. Les caractéristiques du catalyseur ainsi séché et activé sont les suivantes :

| - composition chimique (pondérale) | |
|---|---|
| . fluor | 12,9 % |
| . nickel | 7,4 % |
| . chrome | 5,2 % |

| - propriétés physiques : | |
|---|---|
| . surface BET | 572 m²/g |
| . volume des pores d'un rayon compris entre 4 nm et 63 »m | 0,52 cm³/g |
| . surface des pores de rayon supérieur à 4 nm | 28,5 m²/g |

Le tableau 3 suivant rassemble les conditions opératoires et les résultats obtenus avec les catalyseurs comparatifs C, D et E dans la fluoration du perchloréthylène à pression atmosphérique.

**TABLEAU 3**

| **EXEMPLE** | **3** | **4** | | | **5** | |
|---|---|---|---|---|---|---|
| ***Conditions opératoires*** : | | | | | | |
| - Catalyseur | C | D | D | D | E | E |
| - Température (°C) | 350 | 350 | 300 | 300 | 350 | 320 |
| - Rapport molaire HF/C₂Cl₄ | 6,8 | 7,3 | 7,5 | 7,2 | 6,9 | 8,3 |
| -Temps de contact (s) | 15,9 | 14,9 | 14,0 | 14,7 | 15,6 | 16,9 |
| - Age du catalyseur (h) | 43 | 48 | 291 | 434 | 24 | 48 |

| ***Résultats*** : | | | | | | |
|---|---|---|---|---|---|---|
| - Taux de transformation | | | | | | |
| global de C₂Cl₄ (%) | 60,5 | 95,4 | 89,8 | 68,9 | 50,2 | 35,7 |

| - Sélectivité (% molaire) en : | | | | | | |
|---|---|---|---|---|---|---|
| F125 | 3 | 44,2 | 17 | 5,8 | 2,5 | 1,3 |
| F124 | 23,6 | 13,3 | 33,5 | 29,9 | 5,8 | 4,5 |
| F124a | 0,6 | 0,3 | 1,1 | 2,1 | 2 | 1,4 |
| F123 | 51,4 | 19,1 | 39,8 | 42,2 | 7,1 | 11,3 |
| F123a | 1,4 | 0,1 | 0,7 | 4,1 | 2,9 | 4,7 |
| F122 | 2,9 | 0,1 | 0,8 | 3,3 | 1,7 | 4,6 |
| F121 | 0 | 0 | 0 | 0 | traces | 0,1 |
| F133a | 0,7 | 7,2 | 2,3 | 2,4 | 21,1 | 17 |
| F115 | traces | 9,9 | 0,5 | 0,2 | 3,9 | 2,1 |
| F114 + F 114a | 0,7 | 1,9 | 2,4 | 2,7 | 35 | 27,4 |
| F1111 | 14,4 | 1 | 1,7 | 6,7 | 10,9 | 18 |

La comparaison de ces résultats avec ceux des exemples 1 et 2 effectués dans les conditions opératoires sensiblement identiques montre que :
- le catalyseur C sans chrome (Ex. 3) est nettement moins actif (voir TT_{G} de C₂Cl₄) que le catalyseur mixte Ni-Cr et surtout moins sélectif en F123 + F124 + F125 ;
- le catalyseur D sans nickel (Ex. 4) a une activité initiale comparable à celle du catalyseur mixte Ni-Cr, mais cette activité n'est pas stable dans le temps et, par ailleurs, la sélectivité en F123 + F124 + F125 est moindre ;
- le catalyseur E à support charbon (Ex. 5) est très peu actif et très peu sélectif.

## Revendications

1. Procédé continu de fluoration catalytique du perchloréthylène ou du pentachloréthane en phase gazeuse au moyen d'acide fluorhydrique et d'un catalyseur mixte composé d'oxydes, halogénures et/ou oxyhalogénures de nickel et de chrome déposés sur un support constitué de fluorure d'aluminium ou d'un mélange de fluorure d'aluminium et d'alumine.

2. Procédé selon la revendication 1, caractérisé en ce que la teneur pondérale du catalyseur en nickel et en chrome est comprise entre 0,5 et 20 % pour chaque métal, le rapport atomique nickel/chrome étant compris entre 0,5 et 5, de préférence voisin de 1.

3. Procédé selon la revendication 2, caractérisé en ce que la teneur est comprise entre 2 et 10 % pour chaque métal.

4. Procédé selon l'une des revendications 1 à 3, caractérisé en ce que la température de réaction est comprise entre 200 et 450°C, de préférence entre 250 et 400°C.

5. Procédé selon l'une des revendications 1 à 4, caractérisé en ce que la pression de fonctionnement est comprise entre 0 et 25 bars relatifs.

6. Procédé selon l'une des revendications 1 à 5, caractérisé en ce que le rapport molaire HF/C₂Cl₄ ou C₂HCl₅ est compris entre 1/1 et 20/1, de préférence entre 2/1 et 10/1.

7. Procédé selon l'une des revendications 1 à 6, caractérisé en ce que le temps de contact, calculé dans les conditions réactionnelles, est compris entre 3 et 100 secondes, de préférence entre 5 et 30 secondes et, plus particulièrement, entre 7 et 20 secondes.

8. Procédé selon l'une des revendications 1 à 7, caractérisé en ce que les produits sous-fluorés sont recyclés au réacteur.

9. Procédé selon l'une des revendications 1 à 8, caractérisé en ce que l'on opère en présence d'oxygène.

## Claims

1. Continuous process for catalytic fluorination of perchloroethylene or of pentachloroethane in the gas phase, by means of hydrofluoric acid, and of a mixed catalyst, made up of nickel and chromium oxides, halides and/or oxyhalides deposited on a support consisting of aluminium fluoride or of a mixture of aluminium fluoride and alumina.

2. Process according to Claim 1, characterized in that the weight content of nickel and chromium in the catalyst is between 0.5 and 20 % in the case of each metal, the nickel/chromium atomic ratio being between 0.5 and 5, preferably close to 1.

3. Process according to Claim 2, characterized in that the content is between 2 and 10 % in the case of each metal.

4. Process according to one of Claims 1 to 3, characterized in that the reaction temperature is between 200 and 450°C, preferably between 250 and 400°C.

5. Process according to one of Claims 1 to 4, characterized in that the operating pressure is between 0 and 25 bars gauge.

6. Process according to one of Claims 1 to 5, characterized in that the HF/C₂Cl₄ or C₂HCl₅ molar ratio is between 1/1 and 20/1, preferably between 2/1 and 10/1.

7. Process according to one of Claims 1 to 6, characterized in that the contact time, calculated under the reaction conditions, is between 3 and 100 seconds, preferably between 5 and 30 seconds and, more particularly, between 7 and 20 seconds.

8. Process according to one of Claims 1 to 7, characterized in that the underfluorinated products are recycled to the reactor.

9. Process according to one of Claims 1 to 8, characterized in that the operation is carried out in the presence of oxygen.

## Patentansprüche

1. Kontinuierliches Verfahren zur katalytischen Fluorierung von Perchlorethylen oder Pentachlorethan in der Gasphase mittels Fluorwasserstoff und eines Mischkatalysators, der zusammengesetzt ist aus Nickel- und Chromoxiden, -halogeniden und/oder -oxyhalogeniden, die auf einem Trägermaterial aufgebracht sind, das aus Aluminiumfluorid oder einer Aluminiumfluorid/Aluminiumoxid-Mischung besteht.

2. Verfahren nach Anspruch 1, dadurch gekennzeichnet, daß der Gewichtsanteil des Katalysators an Nickel und Chrom 0,5 bis 20 %, bezogen auf jedes Metall, beträgt, wobei das Atomverhältnis Nickel/Chrom zwischen 0,5 und 5, vorzugsweise bei 1, liegt.

3. Verfahren nach Anspruch 2, dadurch gekennzeichnet, daß der Gehalt 2 bis 10 % für jedes Metall beträgt.

4. Verfahren nach einem der Ansprüche 1 bis 3, dadurch gekennzeichnet, daß die Reaktionstemperatur 200 bis 450 °C, vorzugsweise 250 bis 400 °C, beträgt.

5. Verfahren nach einem der Ansprüche 1 bis 4, dadurch gekennzeichnet, daß der Betriebsdruck 0 bis 25 bar beträgt.

6. Verfahren nach einem der Ansprüche 1 bis 5, dadurch gekennzeichnet, daß das Molverhältnis HF/C₂Cl₄ oder C₂HCl₅ zwischen 1/1 und 20/1, vorzugsweise zwischen 2/1 und 10/1, beträgt.

7. Verfahren nach einem der Ansprüche 1 bis 6, dadurch gekennzeichnet, daß die Kontaktzeit, berechnet unter den Reaktionsbedingungen, 3 bis 100 Sekunden, vorzugsweise 5 bis 30 Sekunden, und insbesondere 7 bis 20 Sekunden, beträgt.

8. Verfahren nach einem der Ansprüche 1 bis 7, dadurch gekennzeichnet, daß die unterfluorierten Produkte dem Reaktor wieder zugeführt werden.

9. Verfahren nach einem der Ansprüche 1 bis 8, dadurch gekennzeichnet, daß man in Anwesenheit von Sauerstoff arbeitet.
